# EUROPEAN PATENT SPECIFICATION

(11) **EP 2 886 654 B1**
(45) Date of publication and mention of the grant of the patent: **19.10.2016**
(21) Application number: 13198723.2
(22) Date of filing: 20.12.2013
(51) Int. Cl.: C12P 5/02, C02F 3/28, C12M 1/107, C02F 11/04

(54) **Method of anaerobic digestion of acidic whey in a four-chamber system**
Verfahren zum anaeroben Abbau von saurer Molke in einem Vier-Kammer-System
Procédé de digestion anaérobie de lactosérum acide dans un système à quatre compartiments

(43) Date of publication of application: 24.06.2015
(73) Proprietor: Rigas Tehniska Universitate, 1658 Riga (LV)
(72) Inventor: Skripsts, Eriks, LV-3113 Plienciems, Engures novads (LV); Rugele, Kristine, LV-1016 Riga (LV); Dubrovskis, Vilis, LV-3913 Iecava (LV); Rubulis, Janis, LV-2130 Dzidrinas, Stopinu novads (LV)
(74) Representative: Vitina, Maruta

(56) References cited:
- EP-A1- 1 553 059
- DE-A1- 19 946 299
- A.N. HASSAN ET AL: "Invited review: Anaerobic fermentation of dairy food wastewater", JOURNAL OF DAIRY SCIENCE, vol. 95, no. 11, 1 November 2012 (2012-11-01), pages 6188-6203, XP055118305, ISSN: 0022-0302, DOI: 10.3168/jds.2012-5732
- GANNOUN H ET AL: "Ecological clarification of cheese whey prior to anaerobic digestion in upflow anaerobic filter", BIORESOURCE TECHNOLOGY, ELSEVIER BV, GB, vol. 99, no. 14, 1 September 2008 (2008-09-01), pages 6105-6111, XP022679171, ISSN: 0960-8524, DOI: 10.1016/J.BIORTECH.2007.12.037 [retrieved on 2008-01-31]
- BREURE A M ET AL: "Protein degradation in anaerobic digestion: influence of volatile fatty acids and carbohydrates on hydrolysis and acidogenic fermentation of gelatin", APPLIED MICROBIOLOGY AND BIOTECHNOLOGY, SPRINGER, BERLIN, DE, vol. 24, no. 5, 1 August 1986 (1986-08-01) , pages 426-431, XP035170662, ISSN: 1432-0614, DOI: 10.1007/BF00294602

## Description

The invention relates to the treatment of dairy by-products and may be used for anaerobic digestion of acidic whey for producing biogas.

Acidic whey is a by-product of the cottage cheese manufacturing, which is obtained after fermentation of milk. Typically acidic whey contains: lactose 44-52 g/L, proteins 6,1-6,6 g/L, fats 0,2-0,3 g/L, minerals 5-7.9 g/L.

It is known that in anaerobic digestion of wastes containing proteins and sugars as major pollutant it should be taken into account that sugars are the preferred substrates for anaerobic organisms. The degradation of proteins becomes inhibited by higher concentrations of lactose [Breure, A.M., Mooijman, K.A., Andel, J.G. Protein degradation in anaerobic digestion: influence of volatile fatty acids and carbohydrates on hydrolysis and acidogenic fermentation of gelatin. Appl. Microbiol. Biotechnol., 24, (1986), p. 426-431]. Therefore, during anaerobic digestion of untreated acidic whey the main problem is that the digestion process of proteins is slower in comparison with the digestion process of lactose that leads to longer common hydraulic retention time (HRT).

The closest prior art is a method of converting and recycling by-products of the dairy industry, in particular whey [Patent application publication EP1553059A1], comprising the following steps:
anaerobic fermentation (digestion) of whey for production of biogas;
recycling of the recovered biogas from the fermentation in a power plant to generate electrical and/or thermal energy;
wherein to enable fermentation of whey prior to said fermentation of whey,
   - the reduction of solids and dissolved substances, comprising calcium, magnesium and phosphate, is carried out increasing pH by the addition of NaOH and
   - proteins are removed. According to the preferred embodiment, before fermentation whey is diluted by water or wastewater and a nitrogen source such as urea is added.

However, the known method is complicated and does not provide anaerobic digestion of wastes rich with proteins. Therefore, the known method is not suitable for small size dairies, which have growing disposal problems and cannot afford high investment costs for whey valorisation technologies (such as whey protein and lactose recovery, spray drying, etc.).

It is known that the two-phase process clearly showed a better performance in management of cheese whey wastewater over the single phase system [Yang K, Yu Y, Hwang S. Selective optimization in thermophilic acidogenesis of cheesewhey wastewater to acetic and butyric acids: partial acidification and methanation. Water Res. 2003; 37(10):2467-77.]. A set of experiments was carried out in laboratory-scale continuously stirred tank reactors A thermophilic two-phase process with the partial acidification followed by a methanation step was operated. Performance of the two-phase process was compared to the single-phase anaerobic system. Maximum rate of COD removal and the rate of methane production in the two-phase process were higher than those of the single-phase system. However, this two-phase process is not yet adapted for practical use for anaerobic digestion of acidic whey for producing biogas.

There is known an apparatus for producing biohydrogen and biomethane from organic substance [Patent LV14431] comprising four sections in the bioreactor, which dimensions are appropriate for the growth rate of microorganisms living in each section. Various micro-organisms of anaerobic digestion are separated in the sections and a better living environment is created for them. Treated organic substance is consecutively moving forward through all chambers. In the bioreactor there is provided a possibility to add in each section catalysts of the corresponding processes, which are necessary for micro-organisms living in each of these sections. However, this method is not appropriate for anaerobic digestion of untreated acidic whey because protein digestion time still remains as a limiting factor.

Technical problem to be solved by the present invention is to create a simple method of anaerobic digestion of untreated acidic whey suitable for small size dairies, using milk proteins as an additional energy source and providing optimal conditions for anaerobic digestion of acidic whey.

A proposed method of anaerobic digestion of acidic whey in a four-chamber system having anaerobic microorganisms for producing biogas comprises the steps of:
injecting untreated acidic whey in the first chamber with organic loading rate 6- 7 kg VS/m^{3.}d adjusting the temperature in the range of 37-38 °C and pH in the range of 4.2-4.5, thus providing the hydrolysis of acidic whey and the sedimentation of at least 90 wt% of milk proteins in the bulk liquid of acidic whey;
separating the milk proteins sediment and 8-12 vol% of the bulk liquid and transferring them to the second chamber adjusting the temperature in the range of 37-38 °C, pH in the range of 7.5-8.1 and hydraulic retention time in the range of 5-7 days, thus providing the treatment and partial anaerobic digestion of the milk proteins in the second chamber;
transferring the remaining bulk liquid from the first chamber to the third chamber and adding the liquid of the treated milk proteins (respectively, the liquid with decomposition products of milk proteins) from the second chamber to the third chamber adjusting in the said third chamber the temperature in the range of 37-38 °C, pH in the range of 6.6-7.2 and hydraulic retention time in the range of 3-4 days and adding nutrient solution and adding nitrogen source being partially provided from said treated milk proteins, thus providing mainly acetogenesis and methanogenesis processes in the third chamber;
transferring effluent from the third chamber to the fourth chamber adding nutrient solution and the source of nitrogen and adjusting in the said fourth chamber the temperature in the range of 37-38 °C, pH in the range of 7.2-7.8 and hydraulic retention time in the range of 3-4 days, thus providing mainly methanogenesis process in the fourth chamber;
during all said steps biogas composition from each chamber is analyzed and collected. The proposed method of anaerobic digestion of acidic whey in the four-chamber system provides full anaerobic digestion of acidic whey, prevents inhibition of protein treatment so making anaerobic digestion process faster. Said method is simple and suitable for small size dairies.

The proposed method preferably is carried out in the four-chamber system, where the volumes of the chambers are balanced with the growth rate of the corresponding group of microorganisms and the proportions of the first, second, third and fourth chambers' volumes are, in % of the total volume of all chambers: 10-15 : 14-18 : 34-40 : 34-40.

It is advisable because the generation time, that is the time required for a population of bacteria to double in size, of methane-forming bacteria is relatively long compared with other anaerobic bacteria.

Preferably FeCl₂ solution is added in the second chamber for the reduction of H₂S because the excessive production of hydrogen sulphide occurs in the second chamber due to the high concentration of sulfur-containing milk proteins that are transferred to the second chamber.

Preferably 5-15 wt% of sludge and 2-10 wt% of digestate are recycled from the fourth chamber to the second chamber, thus providing the optimal biocenosis and nutrient content in the second chamber. Exact recycled quantity depends of the protein content in acidic whey.

Ammonia concentration may be measured by electrical conductivity analysis in the third chamber and in the fourth chamber. These data help to determine the amounts of nitrogen source to be added and the risks of the inhibition.

Preferably urea solution is added as the remaining nitrogen (in addition to nitrogen provided from the treated milk proteins) in the third chamber and as the nitrogen source in the fourth chamber.

Total fatty acids' concentration and total inorganic carbon concentration are measured in the third chamber and in the fourth chamber and the ratio of both concentrations in both said chambers is kept in the range of 0.18-0.40 by dosing of the nutrient and urea solutions. The ratio of total fatty acids' concentration and total inorganic carbon concentration in the range of 0.18-0.40 is optimal for digestion process and fully converting of acidic whey to biogas.

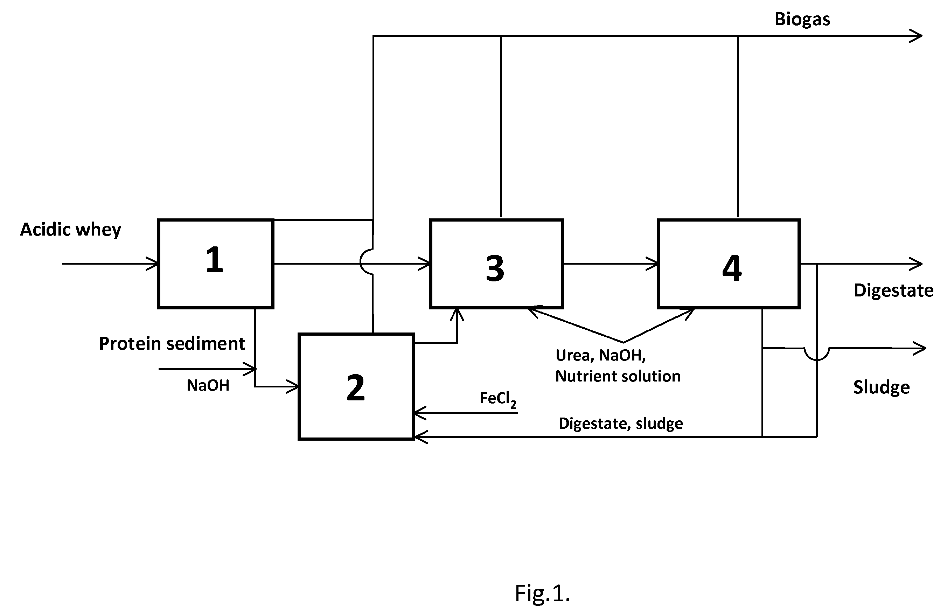

Fig.1 shows the principal technological scheme of anaerobic digestion of acidic whey in a four-chamber system.

The proposed method of anaerobic digestion of acidic whey may be performed as follows. The described example is not the only possible.

The method is carried out in a four-chamber system (Fig.1) having anaerobic microorganisms for producing biogas, where the volumes of the chambers are balanced with the growth rate of the corresponding group of microorganisms and proportions volumes of the first chamber 1, the second chamber 2, the third chamber 3 and the fourth chamber 4 are: 10-15% : 14-18% : 34-40% : 34-40% of the common volume of the chambers. The first chamber 1 is connected with the second chamber 2 and with third chamber 3. The second chamber 2 is also connected with the third chamber 3, which is also connected with the fourth chamber 4.

Acidic whey samples are provided by the dairy, collected in five-litter containers and stored at 4°C for maximum of two weeks to avoid changes of the chemical composition. Acidic whey sample has a high level of total solids (5,68%) and volatile solids (4,92%). Acidic whey has a high strength organic substrate, as COD value was as high as 73,3 g O₂/L, whereas the main component of organic compounds is lactose (48 g/L) and protein content is 7 g/L.

Untreated acidic whey is injected in the first chamber 1 with organic loading rate 6-7 kg VS/m^{3.}d. In the first chamber 1 there is adjusted the temperature in the range of 37-38 °C and pH in the range of 4.2-4.5 (3M NaOH is added if necessary). Such conditions provide the hydrolysis of acidic whey and the sedimentation of at least of 90 wt% of the milk proteins in a bulk liquid of acidic whey.

The sedimented milk proteins and 8-12 vol% of bulk liquid (wet milk proteins' sediment) are separated and transferred to the second chamber 2. In the second chamber 2 there is adjusted the temperature in the range of 37-38 °C and pH in the range of 7.5-8.1 by adding of NaOH. Preferably wet proteins' sediment is transferred to the second chamber 2 together with the corresponding amount of 5M NaOH solution. Such conditions provide partial treatment and anaerobic digestion of the milk proteins. FeCl₂ solution is added for the reduction of H₂S, which is produced in the anaerobic digestion of milk proteins. Hydraulic retention time in the second chamber is in the range of 5-7 days. The said conditions provide mainly methanogenesis process. 5-15% sludge and 2-10% digestate from the fourth chamber 4 are recycled to the second chamber 2 for providing the optimal biocenosis and nutrient content.

After the wet protein sediment is separated, the remaining bulk liquid from the first chamber 1 is transferred to the third chamber 3 and the liquid of the treated milk proteins (respectively, liquid containing decomposition products of milk proteins) from the second chamber 2 are added. In the third chamber 3 there is adjusted the temperature in the range of 37-38 °C. pH in the range of 6.6 - 7.2 is provided by the treated milk proteins from the second chamber 2 and adding 3M NaOH. Nitrogen source is partially provided from the second chamber 2 and additionally the remaining part of nitrogen source is added in the form of urea water solution (5M CO(NH₂)₂, pH 7.5-9.5 at 20 °C). Nutrient solution containing KH₂PO₄ (0.429 g/L), CaCl₂·2H₂O (0.477 g/L), MgCl₂ ·6H₂O (0.151 g/L), FeCl₂·6H₂O (0.0819 g/L), NiCl₂ ·6H₂O (0.0162 g/L), CoCl₂·6H₂O (0.0121 g/L), and ZnCl₂ (0.0417 g/L) is added (in the beginning 2 ml per 1L substrate). Hydraulic retention time is in the range of 3-4 days. The said conditions provide mainly acetogenesis and methanogenesis processes in the third chamber 3.

Effluent from the third chamber 3 is transferred to the fourth chamber 4. In the fourth chamber 4 there is adjusted the temperature in the range of 37-38 °C, pH in the range of 7.2-7.8 by adding 3M NaOH. Nitrogen source is added in the form of urea water solution (5M CO(NH₂)₂, pH 7.5-9.5 at 20 °C). Nutrient solution containing KH₂PO₄ (0.429 g/L), CaCl₂·2H₂O (0.477 g/L), MgCl₂ ·6H₂O (0.151 g/L), FeCl₂·6H₂O (0.0819 g/L), NiCl₂ ·6H₂O (0.0162 g/L), CoCl₂·6H₂O (0.0121 g/L), and ZnCl₂ (0.0417 g/L) is added (in the beginning 2 ml per 1L substrate). Hydraulic retention time is in the range of 3-4 days. The said conditions provide mainly methanogenesis process in the fourth chamber 4.

Total fatty acids' concentration and total inorganic carbon concentration are measured in the third chamber 3 and in the fourth chamber 4 and the ratio of total fatty acids' concentration and total inorganic carbon concentration in both said chambers is kept in the range of 0.18-0.40 by dosing of the said nutrient and urea solutions added in the third chamber 3 and in the fourth chamber 4.

Ammonia concentration is measured by electrical conductivity analysis in the third chamber 3 and in the fourth chamber 4.

During all said steps biogas composition from each chamber is analyzed and collected. Summary composition of biogas is the following: 55-60 % methane and 40-45 % carbon dioxide, H₂S till 200 ppm. Methane yield is in the range of 350-450 L/ kg VS.

Total HRT is 3.5-4 days, which is balanced with the main liquid flow through chambers number: 1,3 and 4.

## Claims

1. A method of anaerobic digestion of acidic whey in a four-chamber system having anaerobic microorganisms for producing biogas comprising the steps of:
injecting untreated acidic whey in the first chamber (1) with organic loading rate 6- 7 kg VS/m^{3.}d, adjusting the temperature in the range of 37-38 °C and pH in the range of 4.2-4.5, thus providing the hydrolysis of acidic whey and the sedimentation of at least 90 wt% of milk proteins in the bulk liquid of acidic whey;
separating the milk proteins sediment and 8-12 vol% of the bulk liquid and transferring them to the second chamber (2), adjusting the temperature in the range of 37-38 °C, pH in the range of 7.5-8.1 and hydraulic retention time in the range of 5-7 days, thus providing the treatment and partial anaerobic digestion of the milk proteins in the second chamber (2);
transferring the remaining bulk liquid from the first chamber to the third chamber (3) and adding the liquid of the treated milk proteins from the second chamber (2) to the third chamber (3), adjusting in the said third chamber the temperature in the range of 37-38 °C, pH in the range of 6.6-7.2 and hydraulic retention time in the range of 3-4 days and adding nutrient solution and adding nitrogen source being partially provided from said treated milk proteins, thus providing mainly acetogenesis and methanogenesis processes in the third chamber (3);
transferring effluent from the third chamber (3) to the fourth chamber (4), adding nutrient solution and the source of nitrogen and adjusting in the said fourth chamber (4) the temperature in the range of 37-38 °C, pH in the range of 7.2-7.8 and hydraulic retention time in the range of 3-4 days, thus providing mainly methanogenesis process in the fourth chamber (4);
wherein, during all said steps biogas composition from each chamber is analyzed and collected.

2. The method according to claim 1, which is carried out in the four-chamber system, where the volumes of the chambers are balanced with the growth rate of the corresponding group of microorganisms and the proportions of volumes of the first chamber (1), the second chamber (2), the third chamber (3) and the fourth chamber (4) are, in % of the total volume of all chambers: 10-15 : 14-18 : 34-40 : 34-40.

3. The method according to claim 1 or 2, in which FeCl₂ solution is added in the second chamber (2) for the reduction of H₂S.

4. The method according to any of the preceding claim, in which 5-15 wt% of sludge and 2-10 wt% of digestate are recycled from the fourth chamber (4) to the second chamber (2), thus providing the optimal biocenosis and nutrient content.

5. The method according to any of the preceding claim, in which ammonia concentration is measured by electrical conductivity analysis in the third chamber (3) and in the fourth chamber (4).

6. The method according to any of the preceding claim, in which urea solution is added as the remaining nitrogen source in the third chamber (3) and as the nitrogen source in the fourth chamber (4).

7. The method according to claim 6, in which total fatty acids' concentration and total inorganic carbon concentration are measured in the third chamber (3) and in the fourth chamber (4) and the ratio of both concentrations in both said chambers is kept in the range of 0.18-0.40 by dosing of the nutrient and urea solutions.

## Patentansprüche

1. Verfahren zum anaeroben Abbau von saurer Molke in einem Vier-Kammer-System mit anaeroben Mikroorganismen zur Erzeugung von Biogas, umfassen folgende Schritte:
Injektion von unbehandelter saurer Molke in der ersten Kammer (1) mit organischer Beladungsrate 6- 7 kg VS/m³.d,
Haltung von Temperatur im Bereich von 37-38 °C und vom pH-Wert im Bereich von 4.2 bis 4.5, um die Hydrolyse von sauren Molken und die Sedimentation der mindestens 90 Gew% des Milchproteins in der Bulk-Flüssigkeit von saurer Molke zu versorgen;
Abtrennung des Milchproteinsediments und 8-12 Vol-% der Flüssigkeitsmasse und dessen Übertragung in die zweite Kammer (2);
Haltung von Temperatur im Bereich von 37-38 ° C, pH im Bereich von 7,5 bis 8,1 und die Einstellung hydraulischer Retentionszeit im Zeitraum von 5-7 Tagen, um die Behandlung und teilweise anaerobe Vergärung des Milchproteins in der zweiten Kammer (2) zu versorgen;
Übertragung der verbleibenden Bulk-Flüssigkeit aus der ersten Kammer in die dritte Kammer (3) und das Hinzufügen der Flüssigkeit der behandelten Milchproteinen aus der zweiten Kammer (2) in die dritte Kammer (3);
Haltung von Temperatur im Bereich von 37-38 ° C, pH im Bereich von 6,6 bis 7,2 und hydraulischer Retentionszeit im Zeitraum von 3-4 Tagen in der genannten dritten Kammer, und die Ergänzung von Nährlösung und das Hinzufügen der Stickstoffquelle, die teilweise von behandelten Milchproteinen bereitgestellt ist, um Acetogenese- und Methanogeneseprozesse in der dritten Kammer (3) zu versorgen;
Übertragung vom Abwasser aus der dritten Kammer (3) in die vierte Kammer (4),
die Ergänzung von Nährlösung und das Hinzufügen der Stickstoffquelle und die Haltung derTemperatur im Bereich von 37-38 ° C, pH im Bereich von 7,2 bis 7,8 und hydraulischer Retentionszeit im Zeitraum von 3-4 Tagen in der genannten vierten Kammer (4), um hauptsächlich den Methanogeneseprozess in der vierten Kammer (4) zu versorgen;
Wobei während aller genannten Schritte die Zusammensetzung von Biogas aus jeder Kammer analysiert und gesammelt wird.

2. Verfahren nach Anspruch 1, das in einem Vier-Kammer-System durchgeführt wird, worin die Volumina der Kammern mit der Wachstumsrate der entsprechenden Gruppe von Mikroorganismen ausgeglichen werden und die Proportionen der Volumina der ersten Kammer (1), der zweiten Kammer (2), der dritten Kammer (3) und der vierten Kammer (4) sind, in % des Gesamtvolumens aller Kammern:
10-15: 14-18: 34-40: 34-40.

3. Verfahren nach Anspruch 1 oder 2, worin FeCl₂ Lösung in der zweiten Kammer (2) zur Reduktion von H₂S zugesetzt wird.

4. Verfahren nach einem der vorhergehenden Ansprüche, worin 5-15 Gew% vom Schlamm und 2-10 Gew% vom Gärrest aus der vierten Kammer (4) in die zweite Kammer (2) zurückgeführt sind, wodurch die optimale Biocenosis und der Nährstoffgehalt versorgt sind.

5. Verfahren nach einem der vorhergehenden Ansprüche, worin Ammoniakkonzentration durch die elektrische Leitfähigkeitsanalyse in der dritten Kammer (3) und in der vierten Kammer (4) gemessen wird.

6. Verfahren nach einem der vorhergehenden Ansprüche, worin Harnstofflösung als die verbleibenden Stickstoffquelle in der dritten Kammer (3) und als Stickstoffquelle in der vierten Kammer (4) zugegeben wird.

7. Verfahren nach Anspruch 6, worin die gesamte Fettsäurenkonzentration und die gesamte Konzentration vom anorganischen Kohlenstoff in der dritten Kammer (3) und in der vierten Kammer (4) gemessen werden und das Verhältnis bei der Konzentrationen in beiden genannten Kammern im Bereich von 0,18 bis 0,40 bei Dosierung von Nährstoff- und Harnstofflösungen gehalten wird.

## Revendications

1. Une méthode de digestion anaérobie de lactosérum acide dans un système de quatre chambres ayant les micro-organismes anaérobies pour produire le biogaz comprenant les pas suivants:
l'injection de lactosérum acide non traitée dans la première chambre (1) avec le taux de chargement organique 6 - 7 kg VS/m³.d,
en réglant la température de l'ordre de 37-38 °C et de ph de l'ordre de 4.2-4.5, en fournissant ainsi l'hydrolyse de lactosérum acide et la sédimentation d'au moins 90 % en poids de protéines du lait dans le liquide en gros de lactosérum acide;
en séparant le sédiment de protéines de lait et le 8-12 vol% du liquide en gros et en les transférant à la deuxième chambre (2),
en réglant la température de l'ordre de 37-38 °C, le pH de l'ordre de 7.5-8.1 et le temps de rétention hydraulique de l'ordre de de 5 à 7 jours, permettant ainsi le traitement et la digestion anaérobie partielle des protéines de lait dans la deuxième chambre (2);
en transférant le liquide en gros restant de la première chambre à la troisième chambre (3) et en ajoutant le liquide des protéines de lait traitées de la deuxième chambre (2) à la troisième chambre (3),
en réglant dans dite troisième chambre la température de l'ordre de 37-38 °C, le ph de l'ordre de 6.6-7.2 et le temps de rétention hydraulique de l'ordre de 3 à 4 jours et en ajoutant la solution nutritive et en ajoutant la source d'azote étant partiellement fournie des protéines de lait traitées, en fournissant ainsi principalement le procesus d'acetogenesis et de methanogenesis dans la troisième chambre (3);
en transférant l'effluent de la troisième chambre (3) à la quatrième chambre (4),
en ajoutant la solution nutritive et la source d'azote et en réglant dans la quatrième chambre (4) la température de l'ordre de 37-38 °C, le pH de l'ordre de 7.2-7.8 et le temps de rétention hydraulique de l'ordre de 3 à 4 jours, en fournissant ainsi principalement le procesus de méthanogénèse dans la quatrième chambre (4);
où la composition de biogaz de chaque chambre est analysée et recueillie pendant toutes ces étapes mentionnées.

2. La méthode selon la revendication 1, qui est réalisée dans le système de quatre chambres, où les volumes des chambres sont équilibrés avec le taux de croissance du groupe correspondant de micro-organismes et des proportions de volumes de la première chambre (1), la deuxième chambre (2), la troisième chambre (3) et la quatrième chambre (4) en % du volume total de toutes les chambres sont:
10-15: 14-18 : 34-40 : 34-40.

3. La méthode selon la revendication 1 ou 2, dans laquelle la solution FeCl₂ est ajoutée dans la deuxième chambre (2) pour la réduction de H₂S.

4. La méthode selon une des revendications précédentes, dans laquelle % de poids est de 5 à 15 de vase et de 2-10 de digestate et qui sont recyclés de la quatrième chambre (4) à la deuxième chambre (2), en fournissant ainsi le biocenosis et le contenu nutritif optimaux.

5. La méthode selon une des revendications précédentes, dans laquelle la concentration d'ammoniaque est mesurée par l'analyse de conductivité électrique dans la troisième chambre (3) et dans la quatrième chambre (4).

6. La méthode selon une des revendications précédentes, dans laquelle la solution d'urée est ajoutée comme la source d'azote restante dans la troisième chambre (3) et comme la source d'azote dans la quatrième chambre (4).

7. La méthode selon la revendication 6, dans laquelle la concentration totale d'acides gras et la concentration totale de carbone inorganique sont mesurées dans la troisième chambre (3) et dans la quatrième chambre (4) et le rapport de deux concentrations dans ces deux chambres mentionnés est gardée dans la gamme de 0.18-0.40 en dosant de solution nutritive et des solution d'urée.
